# EUROPEAN PATENT APPLICATION

(11) **EP 2 292 609 A1**
(43) Date of publication of application: **09.03.2011**
(21) Application number: 10170757.8
(22) Date of filing: 26.07.2010
(51) Int. Cl.: C07D 249/04, A61K 31/4192, A61P 25/00

(54) **Crystalline forms of rufinamide**

(30) Priority: 04.08.2009 IT MI20091412
(71) Applicant: Dipharma Francis S.r.l., 20021 Baranzate (MI) (IT)
(72) Inventor: Razzetti, Gabriele, 20099 Sesto San Giovanni (MI) (IT); Vladiskovic, Chiara, 20146 Milano (IT); Allegrini, Pietro, 20097 S. Donato Milanese (MI) (IT)
(74) Representative: Minoja, Fabrizio

(57) **Abstract**

Disclosed is a crystalline form of rufinamide selected from:
• a substantially anhydrous and approximately monosolvated with trifluoroacetic acid crystalline form, hereinafter designated as Form a, and
• a crystalline form, hereinafter designated as Form β, characterised by an XRPD spectrum as shown in Figure 3, wherein the most intense peaks fall at 4.5, 9.0, 13.5, 18.0, 18.8, 19.5, 20.6, 24.6, 25.7, 26.5, 27.4, 27.9, 28.7, 30.0 and 31.8 ± 0.2° in 2θ.

## Description

### FIELD OF INVENTION

The present invention relates to two novel crystalline forms of 1-[(2,6-difluorophenyl)methyl]-1*H*-1,2,3-triazole-4-carboxyamide (rufinamide), in particular a form solvated with trifluoroacetic acid and an anhydrous form.

### PRIOR ART

Rufinamide, or 1-[(2,6-difluorophenyl)methyl]-1*H*-1,2,3-triazole-4-carboxyamide, is a triazole with antiepileptic activity which is particularly useful to treat Lennox-Gastaut syndrome. Rufinamide is known from EP 199262. WO 98/56772 reports various crystalline forms of rufinamide, in particular forms designated as A, A', B and C.

The known crystalline forms of rufinamide do not possess optimum characteristics for the preparation of pharmaceutical formulations. For example, form A, which is more stable than the other known crystalline forms and is the one present on the market, has a low apparent density, poor flowability and a strong tendency to form agglomerates.

Crystalline forms of rufinamide are therefore required, including hydrated or solvated forms, which present advantages compared with the known forms, such as better bioavailability, and which are also more suitable for the preparation of pharmaceutical formulations.

### SUMMARY OF THE INVENTION

The present invention relates to two new crystalline forms of 1-[(2,6-difluorophenyl)methyl]-1*H*-1,2,3-triazole-4-carboxyamide, in particular a form thereof solvated with trifluoroacetic acid, hereinafter designated as Form α and an anhydrous form thereof, hereinafter designated as Form β.

### BRIEF DESCRIPTION OF FIGURES AND ANALYSIS METHODS

The different crystalline modifications of rufinamide were characterised by X-ray powder diffraction (XRPD), ¹H-NMR Nuclear Magnetic Resonance spectrometry, differential scanning calorimetry (DSC), and infra-red spectrophotometry (FT-IR). The water content of the compounds was determined by titration with the Karl Fischer technique. The X-ray diffraction spectra (XRPD) were collected with the APD-2000 automatic powder and liquid diffractometer, manufactured by Ital-Structures, under the following operating conditions: Bragg-Brentano geometry, CuKα radiation (λ = 1,5418 Å), scanning with a 2θ angle range of 3-40° and a step size of 0.03°, for a time of 1 sec. The ¹H-NMR spectra were acquired with a Varian Mercury 300 spectrometer, using DMSO-d6 as solvent. The DSC thermograms were acquired with a Mettler-Toledo DSC 822e differential scanning calorimeter, under the following operating conditions: open aluminium capsule, range 30-300°C at the rate of 10°C/min, with nitrogen as purge gas (80 ml/min). The IR spectra were recorded with a Perkin-Elmer Paragon 500 spectrophotometer for 16 scans between 4000 and 650 cm⁻¹.
Figure 1: XRPD spectrum of rufinamide Form α
Figure 2: DSC thermogram of rufinamide Form α
Figure 3: XRPD spectrum of rufinamide Form β
Figure 4: DSC thermogram of rufinamide Form β
Figure 5: FT-IR spectrum of rufinamide Form β

The particle size and D₅₀ are determined by the well-known laser light scattering technique, using a Malvern Mastersizer MS1 instrument under the following operating conditions:
- 300RF mm lens with 2.4 mm laser beam length;
- 500 mg sample dispersed in 10 ml of hexane (ACS reagent) with 1% of SPAN 85^{®}, without pre-sonication, and with a stirring rate of 2500 rpm.

### DETAILED DESCRIPTION OF THE INVENTION

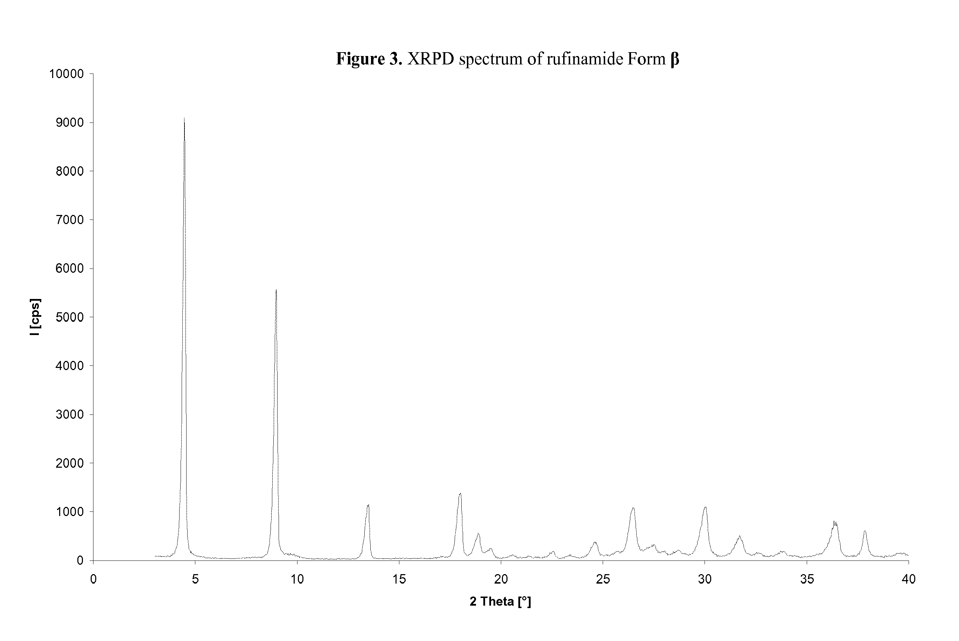

The object of the present invention is a crystalline form of rufinamide selected from:
- a substantially anhydrous and approximately monosolvated with trifluoroacetic acid crystalline form, hereinafter designated as Form α, and
- a crystalline form, hereinafter designated as Form β, characterised by an XRPD spectrum as shown in Figure 3, wherein the most intense peaks fall at 4.5, 9.0, 13.5, 18.0, 18.8, 19.5, 20.6, 24.6, 25.7, 26.5, 27.4, 27.9, 28.7, 30.0 and 31.8 ± 0.2° in 2θ.

Rufinamide in crystalline Form α has a water content of between 0 and approximately 1% w/w, preferably between approximately 0.1 and 0.5%, and can therefore be defined as substantially anhydrous.

This form also has a trifluoroacetic acid content of between approximately 15% and approximately 50% w/w, preferably between approximately 20 and approx. 40% w/w, and more preferably around approximately 30% w/w, and can therefore be defined as approximately monosolvated.

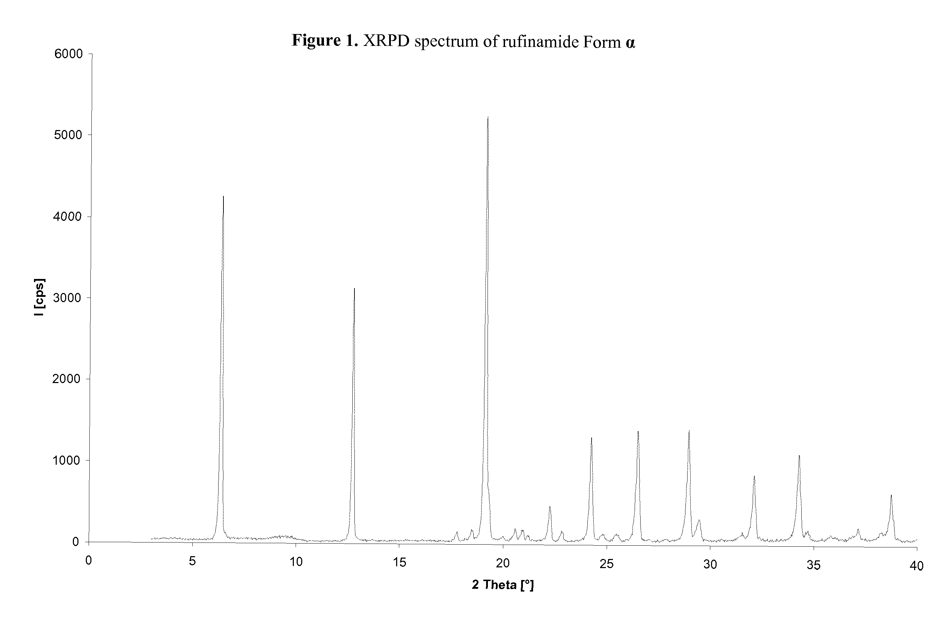

Rufinamide in crystalline Form α is also characterised by an XRPD spectrum, as shown in Figure 1, where the most intense peaks fall at 6.4, 12.7, 17.7, 18.5, 19.1, 22.2, 22.8, 24.2, 26.5, 28.9, 29.4, 32.1 and 34.3 ± 0.2° in 2θ.

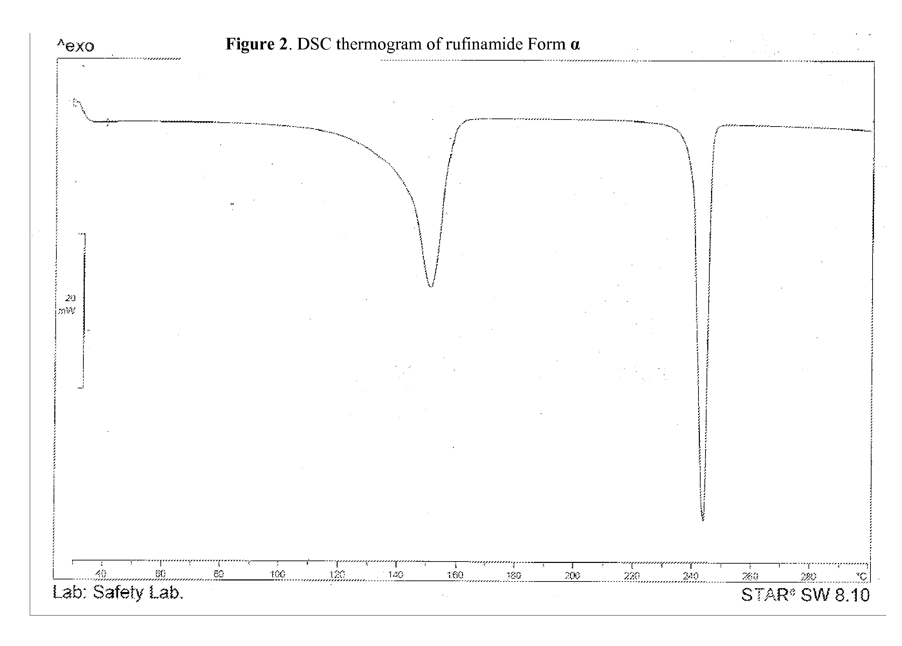

Rufinamide in crystalline Form α is also characterised by a DSC thermogram in which the main thermal events are found between approximately 130 and approximately 150°C (broad band, endothermy, loss of trifluoroacetic acid) and at approximately 238-240°C (sharp peak, endothermy, fusion), as shown in Figure 2.

Rufinamide in crystalline Form β has a water content of between 0 and approximately 1%, preferably between approximately 0.1 and 0.5%, and can therefore be defined as substantially anhydrous.

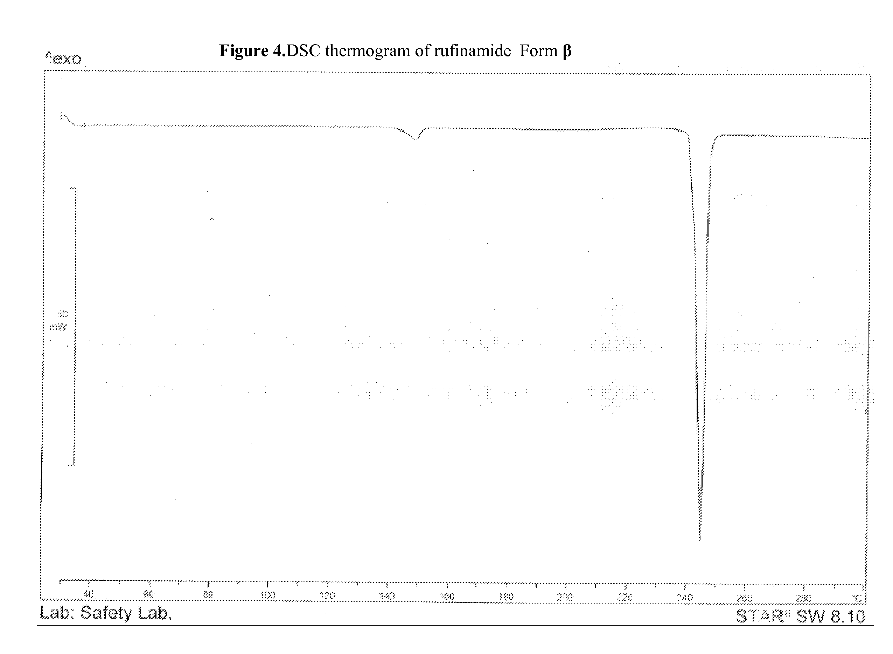

Said Form β is characterised by a DSC thermogram in which the main thermal events are found at approximately 135-150°C (endothermy) and at approximately 238-240°C (sharp peak, endothermy, fusion), as shown in Figure 4.

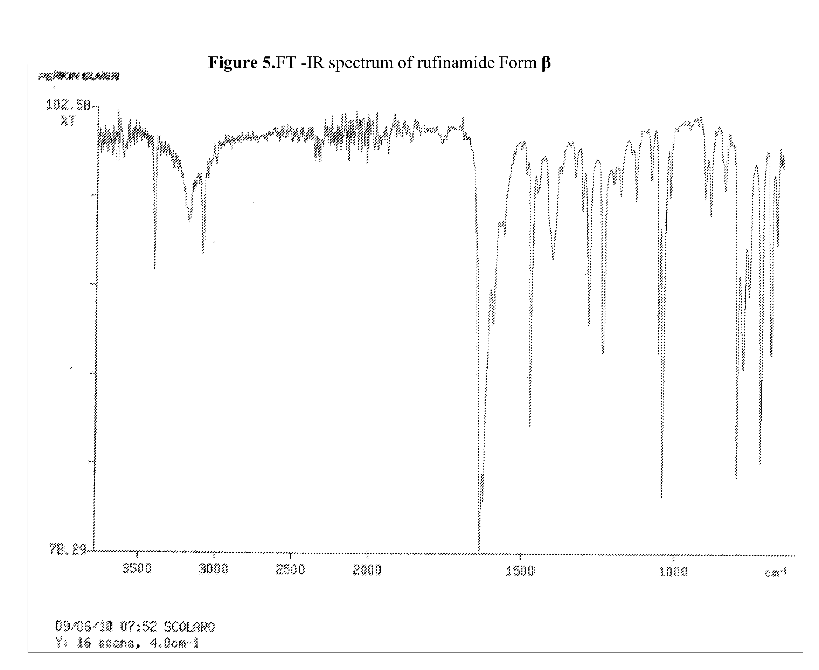

Said crystalline Form β is also characterised by an FT-IR spectrum wherein the main bands fall at 3408, 3181, 3087, 1635, 1626, 1596, 1472, 1449, 1402, 1328, 1304, 1283, 1236, 1203, 1180, 1131, 1080, 1054, 1041, 1019, 904, 887, 839, 798, 778, 761, 722, 688 and 668 cm⁻¹, as shown in Figure 5.

Rufinamide Form α, as defined herein, can be prepared by a process comprising:
- providing a dispersion of rufinamide in trifluoroacetic acid or a mixture thereof with another solvent, and dissolution thereof;
- cooling of the solution to precipitate crystalline form α; and
- recovering of the solid.

Any known crystalline form of rufinamide can be used as starting material.

The trifluoroacetic acid can be mixed with another solvent, for example one to three solvents, preferably one, selected from an ether, for example tetrahydrofuran (THF); a C₁-C₆ straight or branched alkanol, preferably a C₁-C₄ alkanol such as methanol, ethanol or isopropanol; a ketone, such as acetone or methyl ethyl ketone; an alkyl ester, such as methyl acetate, ethyl acetate or isopropyl acetate; an aliphatic or aromatic hydrocarbon such as hexane, heptane, toluene or xylene; a carboxylic acid, such as formic, acetic or propionic acid; and a polar protic solvent, such as water.

The concentration of rufinamide in the starting dispersion can be between approximately 2% and 50% w/w, preferably between approximately 10% and 35% w/w.

If desired, rufinamide can be dissolved in the starting dispersion by heating it to a temperature of between approximately 25°C and the reflux temperature of trifluoroacetic acid or of the mixture of solvents, and more preferably between approximately 80 and 100°C.

The solution can be cooled, to precipitate crystalline Form α, to a temperature of between approximately -10 and approximately 70°C, in particular around 0°C; preferably at a gradient of approximately 2-20°C / minute.

If desired, precipitation can be promoted by triggering crystallisation by adding crystals of rufinamide in crystalline Form α, previously obtained.

Solid rufinamide in crystalline Form α can be recovered by known techniques such as filtration or centrifugation, preferably by filtration through a Bückner filter.

The size of the crystals of rufinamide Form α thus obtained is characterised by a D₅₀ value of between approximately 25 and approximately 250 µm. If desired, said value can be reduced by micronisation or fine grinding.

Rufinamide Form β, as defined herein, can be obtained by a process comprising:
- treatment of rufinamide Form α with a solvent to convert it to crystalline Form β; and
- recovery of the solid.

The solvent used can be a solvent or mixture of two or three solvents, preferably two, in which rufinamide Form α is insoluble or poorly soluble, such one of those mentioned above, but in which trifluoroacetic acid is soluble, except a carboxylic acid.

If desired, the treatment can be performed in the presence of a base.

The base can be ammonia, an alkali metal hydroxide or an alkali metal C₁-C₆ alkoxide, for example of sodium or potassium, preferably sodium or potassium hydroxide, sodium or potassium methoxide, or sodium or potassium ethoxide; an alkali metal carbonate or bicarbonate salt such as potassium carbonate or sodium bicarbonate; or an organic amine such as triethylamine. The base used is preferably ammonia in aqueous solution, preferably around 30-35% w/w.

Treatment with the solvent is typically conducted by adding rufinamide Form α to the solvent or vice versa, typically slowly and under cooling, for example at a temperature of between approximately 10°C and 0°C, preferably around 0°C.

The addition is typically performed in a time of between approximately 20 and 90 minutes, preferably between approximately 30 minutes and 45 minutes.

The solid can be recovered by known methods, such as centrifugation or filtration, preferably filtration through a Bückner filter.

After filtration the solid can be washed with a solvent or a mixture of solvents in which rufinamide Form β is insoluble, such as a C₁-C₆ straight or branched alkanol, preferably a C₁-C₄ alkanol such as methanol, ethanol or isopropanol, to remove any excess of the solvent used and/or of the base, such as water and/or residual ammonia, used in the treatment of Form α.

The size of the crystals of rufinamide Form α obtained in accordance with the present process is characterised by a D₅₀ value of between approximately 25 and approximately 250 µm. If required, said value can be reduced by micronisation or fine grinding.

Rufinamide Form α and Form β, obtained by the process according to the invention, have a purity equal to or greater than 99%, preferably equal to or greater than 99.8%.

The novel rufinamide forms α and β are useful for the administration of rufinamide in mammals in the need of said treatment. They can be administered alone or in combination or in admixture with one or more known polymorphic forms of rufinamide, for example those designated as A, A', B and C mentioned above. The content of each form in the mixtures depends on their physical and biological properties and will be determined by the skilled person. A variety of pharmaceutical compositions can be prepared for the administration in humans or animals, according to known techniques. The amount of rufinamide in capsules, tablets, sugar-coated pills or other forms for the single oral administration may range from about 150 to about 450 mg per dose unit. Therefore, the invention also provides a pharmaceutical composition comprising, as active ingredient, rufinamide Form α or Form β, or a mixture thereof, or a mixture of at least one of them with one or more known polymorphic forms of rufinamide, in admixture with a suitable carrier and/or excipient.

The following examples illustrate the invention.

### Example 1. Preparation of rufinamide Form α

5.0 g of rufinamide are suspended in 20 ml of trifluoroacetic acid and 10 ml of water. The mixture is then heated to approximately 95°C until the solid is completely dissolved. It is then cooled slowly to 0°C. A white solid precipitates, which is recovered by filtration through a Bückner filter.

XRPD: Main peaks at 6.4, 12.7, 17.7, 18.5, 19.1, 22.2, 22.8, 24.2, 26.5, 28.9, 29.4, 32.1 and 34.3 ± 0.2° in 2θ.

H¹-NMR (in DMSO): *s* (1H), 8.51 ppm; broad *s* (1H), 7.8 ppm; *m* (2H), 7.4-7.6 ppm; *m* (2H), 7.16 ppm; *s* (2H), 5.70 ppm.

### Example 2. Preparation of rufinamide Form β

1.5 g of rufinamide Form α are added slowly to a solution cooled to 0°C consisting of 3 ml of water and 3 ml of 33% (w/w) aqueous ammonia. The solid is recovered by filtration through a Bückner filter.

XRPD: Main peaks at 4.5, 9.0, 13.5, 18.0, 18.8, 19.5, 20.6, 24.6, 25.7, 26.5, 27.4, 27.9, 28.7, 30.0 and 31.8 ± 0.2° in 2θ.

H¹-NMR (in DMSO): *s* (1H), 8.51 ppm; broad *s* (1H), 7.8 ppm; *m* (2H), 7.4-7.6 ppm; *m* (2H), 7.16 ppm; *s* (2H), 5.70 ppm.

### Example 3. Preparation of rufinamide Form α

4.0 g of rufinamide are suspended in 8 ml of trifluoroacetic acid. The mixture is then heated to reflux until the solid is completely dissolved. It is then cooled slowly to 0°C. A white solid precipitates, which is recovered by filtration through a Bückner filter.

XRPD: Main peaks at 6.4, 12.7, 17.7, 18.5, 19.1, 22.2, 22.8, 24.2, 26.5, 28.9, 29.4, 32.1 and 34.3 ± 0.2° in 2θ.

H¹-NMR (in DMSO): *s* (1H), 8.51 ppm; broad *s* (1H), 7.8 ppm; *m* (2H), 7.4-7.6 ppm; *m* (2H), 7.16 ppm; *s* (2H), 5.70 ppm.

### Example 4. Preparation of rufinamide Form β

10 g of rufinamide are dispersed in 30 ml of trifluoroacetic acid and 16 ml of water. The mixture is heated at approximately 95°C until the solid is completely dissolved. It is then slowly cooled to 0°C, and the solid precipitate is filtered. This solid is then added slowly to a solution of 20 ml water and 20 ml of 33% (w/w) aqueous ammonia, and filtered again. The solid is washed twice on the filter with isopropanol, once with a 1:1 mixture of isopropanol:heptane, and twice with heptane.

XRPD: Main peaks at 4.5, 9.0, 13.5, 18.0, 18.8, 19.5, 20.6, 24.6, 25.7, 26.5, 27.4, 27.9, 28.7, 30.0 and 31.8 ± 0.2° in 2θ.

H¹-NMR (in DMSO): *s* (1H), 8.51 ppm; broad *s* (1H), 7.8 ppm; *m* (2H), 7.4-7.6 ppm; *m* (2H), 7.16 ppm; *s* (2H), 5.70 ppm.

### Example 5. Preparation of rufinamide Form α

500 mg of rufinamide are suspended in 4 ml of toluene, and the mixture is heated to approximately 80°C. Trifluoroacetic acid is added drop by drop until the solid has completely dissolved. The solution is then slowly cooled to room temperature. A white solid precipitates, which is recovered by filtration through a Bückner filter.

XRPD: Main peaks at 6.4, 12.7, 17.7, 18.5, 19.1, 22.2, 22.8, 24.2, 26.5, 28.9, 29.4, 32.1 and 34.3 ± 0.2° in 2θ.

H¹-NMR (in DMSO): *s* (1H), 8.51 ppm; broad *s* (1H), 7.8 ppm; *m* (2H), 7.4-7.6 ppm; *m* (2H), 7.16 ppm; *s* (2H), 5.70 ppm.

## Claims

1. A crystalline form of rufinamide selected from:
• a substantially anhydrous and approximately monosolvated with trifluoroacetic acid crystalline form, hereinafter designated as Form α, and
• a crystalline form, hereinafter designated as Form β, **characterised by** an XRPD spectrum as shown in Figure 3, wherein the most intense peaks fall at 4.5, 9.0, 13.5, 18.0, 18.8, 19.5, 20.6, 24.6, 25.7, 26.5, 27.4, 27.9, 28.7, 30.0 and 31.8 ± 0.2° in 2θ.

2. Crystalline Form α as claimed in claim 1, having a trifluoroacetic acid content of between approximately 15% and approximately 50% w/w, preferably between approximately 20% and approximately 40% w/w.

3. Crystalline Form α as claimed in claim 1 or 2, **characterised by** a DSC thermogram wherein the main thermal events are found between approximately 130 and approximately 150°C, and at approximately 238-240°C.

4. Crystalline Form α as claimed in claims 1 to 3, **characterised by** an XRPD spectrum, as shown in Figure 1, wherein the most intense peaks fall at 6.4, 12.7, 17.7, 18.5, 19.1, 22.2, 22.8, 24.2, 26.5, 28.9, 29.4, 32.1 and 34.3 ± 0.2° in 2θ.

5. Crystalline Form β as claimed in claim 1, **characterised by** an FT-IR spectrum wherein the main bands fall at 3408, 3181, 3087, 1635, 1626, 1596, 1472, 1449, 1402, 1328, 1304, 1283, 1236, 1203, 1180, 1131, 1080, 1054, 1041, 1019, 904, 887, 839, 798, 778, 761, 722, 688 and 668 cm⁻¹; as shown in Figure 5.

6. Process for the preparation of rufinamide Form α, as defined in claim 1, comprising:
• providing a dispersion of rufinamide in trifluoroacetic acid or a mixture thereof with another solvent, and dissolution thereof;
• cooling of the solution to precipitate crystalline Form α; and
• recovering of the solid.

7. A process as claimed in claim 6, wherein the mixture with another solvent is a mixture containing one to three solvents selected from an ether, a C₁-C₆ straight or branched alkanol, a ketone, an alkyl ester, an aliphatic or aromatic hydrocarbon, a carboxylic acid and a polar protic solvent.

8. A process as claimed in claim 6, wherein the solution is cooled, to precipitate crystalline Form α, to a temperature of between approximately -10 and approximately 70°C, in particular around 0°C.

9. Process for the preparation of rufinamide Form β, as defined in claim 1, comprising:
• treatment of rufinamide Form α, as claimed in claim 1, with a solvent, to convert it to crystalline Form β; and
• recovery of the solid.

10. A process as claimed in claim 9, wherein the solvent is a solvent, or a mixture of two or three solvents, in which rufinamide Form α is insoluble or poorly soluble, but in which trifluoroacetic acid is soluble, except a carboxylic acid.

11. A process as claimed in claim 9, wherein the treatment is performed in the presence of a base.

12. A process as claimed in claim 11, wherein the base is ammonia, an alkali metal hydroxide or an alkali metal C₁-C₆ alkoxide, an alkali metal carbonate salt, an alkali metal bicarbonate salt, or an organic amine; preferably ammonia in aqueous solution, in particular around 30-35% w/w.

13. A pharmaceutical composition comprising, as active ingredient, rufinamide Form α or Form β, as defined in claim 1, or a mixture thereof, or a mixture of at least one of them with one or more known polymorphic forms of rufinamide, in admixture with a suitable carrier and/or excipient.
